# EUROPÄISCHE PATENTSCHRIFT

(11) **EP 3 272 729 B1**
(45) Veröffentlichungstag und Bekanntmachung des Hinweises auf die Patenterteilung: **04.09.2019**
(21) Anmeldenummer: 16180053.7
(22) Anmeldetag: 19.07.2016
(51) Int. Cl.: C07C 67/37, C07C 69/24, C07F 9/58

(54) **VERFAHREN ZUR HERSTELLUNG VON ESTERN DURCH CARBONYLIERUNG VON ETHERN**
METHOD FOR THE PREPARATION OF ESTERS THROUGH THE CARBONYLATION OF ETHERS
PROCÉDÉ DE FABRICATION D'ESTER PAR CARBONYLATION D'ESTER

(43) Veröffentlichungstag der Anmeldung: 24.01.2018
(73) Patentinhaber: Evonik Degussa GmbH, 45128 Essen (DE)
(72) Erfinder: DONG, Kaiwu, 236800 Bo Zhou (CN); JACKSTELL, Ralf, 27478 Cuxhaven Altenwalde (DE); NEUMANN, Helfried, 18055 Rostock (DE); BELLER, Matthias, 18211 Ostseebad Nienhagen (DE); FRIDAG, Dirk, 45721 Haltern an See (DE); HESS, Dieter, 45770 Marl (DE); DYBALLA, Katrin Marie, 45657 Recklinghausen (DE); GEILEN, Frank, 45721 Haltern am See (DE); FRANKE, Robert, 45772 Marl (DE)

(56) Entgegenhaltungen:
- WO-A1-01/47861
- WO-A1-2006/121778
- WO-A1-2011/083305
- GB-A- 651 853
- US-B1- 6 335 471

## Beschreibung

Die vorliegende Erfindung betrifft ein neues Verfahren zur Herstellung von Estern durch Carbonylierung von Ethern.

Die Alkoxycarbonylierung ethylenisch ungesättigter Verbindungen ist ein bekanntes Verfahren zur Herstellung von Estern. Bei diesem Verfahren werden ethylenisch ungesättigte Verbindungen (Olefine) mit Kohlenmonoxid und Alkoholen in Gegenwart eines Metall-Ligand-Komplexes zu den entsprechenden Estern umgesetzt. Üblicherweise wird als Metall Palladium eingesetzt. Das folgende Schema zeigt die allgemeine Reaktionsgleichung einer Alkoxycarbonylierung:

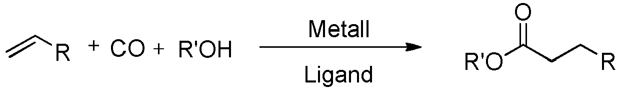

Ein sehr gutes katalytisches System für dieses Verfahren wurde von Lucite - jetzt Mitsubishi Rayon - entwickelt und verwendet einen Liganden auf Basis von 1,2-Bis(di-tert-butylphosphinomethyl)benzol (DTBPMB) (W. Clegg, G. R. Eastham, M. R. J. Elsegood, R. P. Tooze, X. L. Wang, K. Whiston, Chem. Commun 1999, 1877-1878).

In der WO 01/47861 A1 wird ein Verfahren zur Herstellung von Estern beschrieben. Hierbei wird ein Olefin oder Ether mit einem Alkohol in Gegenwart von CO und BF₃*2ROH umgesetzt.

In der WO 2006/121778 A1 wird ein Verfahren zur Herstellung eines Esters beschreiben. Hierbei wird eine Carbonsäure mit CO in Gegenwart eines Katalysators, welcher Mordenite oder Ferrite umfasst, umgesetzt.

In der GB 651 853 A wird ein Verfahren zur Herstellung eines Esters beschreiben. Hierbei wird ein nicht-aromatisches Olefin mit CO und einem primären Alkohol in Gegenwart eines Kobaltsalzes umgesetzt.

Es ist bislang nicht bekannt, die Alkoxycarbonylierung ausgehend von anderen Rohstoffen als ethylenisch ungesättigten Verbindungen durchzuführen. Es ist deshalb Aufgabe der vorliegenden Erfindung, ein Verfahren zur Herstellung von Estern bereit zu stellen, bei dem als Ausgangsprodukt an Stelle von ethylenisch ungesättigten Verbindungen andere Rohstoffe verwendet werden. Von besonderem Interesse ist dabei der Einsatz von Ethern aus Ausgangsprodukt.

Es hat sich überraschenderweise gezeigt, dass diese Aufgabe durch ein Verfahren gelöst wird, bei dem Ether mit CO in Gegenwart besonderer Benzol-basierter Diphosphinliganden, bei denen mindestens eine Phosphingruppe mit einem Heteroarylrest substituiert ist, direkt zu Estern umgesetzt werden. Das erfindungsgemäße Verfahren zeichnet sich dadurch aus, dass der Reaktion kein Alkohol zugesetzt werden muss. Somit bietet die Erfindung einen kostengünstigen und einfachen Weg zur Darstellung von Estern, der gegenüber der herkömmlichen Alkoxycarbonylierung nicht nur die Verwendung von Ethern als Ausgangsprodukt ermöglicht, sondern auch den Einsatz von Alkoholen als weiteres Edukt überflüssig macht.

Die Erfindung betrifft somit ein Verfahren umfassend die Verfahrensschritte:
a) Vorlegen eines Ethers mit 3 bis 30 Kohlenstoffatomen;
b) Zugabe eines Phosphinliganden und einer Verbindung, welche Pd umfasst,
   oder Zugabe eines Komplexes umfassend Pd und einen Phosphinliganden;
b') Zugabe einer Säure zum Reaktionsgemisch;
c) Zuführen von CO;
d) Erwärmen des Reaktionsgemisches, wobei der Ether zu einem Ester umgesetzt wird; wobei der Phosphinligand eine Verbindung gemäß Formel (**I**) ist 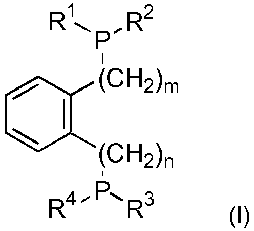 wobei
   m und n unabhängig voneinander jeweils 0 oder 1 sind;
   R¹, R², R³, R⁴ jeweils unabhängig voneinander ausgewählt sind aus -(C₁-C₁₂)-Alkyl, -(C₃-C₁₂)-Cycloalkyl, -(C₃-C₁₂)-Heterocycloalkyl, -(C₆-C₂₀)-Aryl, -(C₃-C₂₀)-Heteroaryl;
   mindestens einer der Reste R¹, R², R³, R⁴ für einen -(C₃-C₂₀)-Heteroarylrest steht; und
   R¹, R², R³, R⁴, falls diese für -(C₁-C₁₂)-Alkyl, -(C₃-C₁₂)-Cycloalkyl, -(C₃-C₁₂)-Heterocycloalkyl,-(C₆-C₂₀)-Aryl, oder -(C₃-C₂₀)-Heteroaryl stehen,
   jeweils unabhängig voneinander mit einem oder mehreren Substituenten ausgewählt aus -(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -S-(C₁-C₁₂)-Alkyl, -S-(C₃-C₁₂)-Cycloalkyl, -COO-(C₁-C₁₂)-Alkyl, -COO-(C₃-C₁₂)-Cycloalkyl, -CONH-(C₁-C₁₂)-Alkyl, -CONH-(C₃-C₁₂)-Cycloalkyl,-CO-(C₁-C₁₂)-Alkyl, -CO-(C₃-C₁₂)-Cycloalkyl, -N-[(C₁-C₁₂)-Alkyl]₂, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, -COOH, -SO₃H, -NH₂, Halogen substituiert sein können; und wobei
   dem Reaktionsgemisch kein Alkohol zugesetzt wird.

Hierbei können die Verfahrensschritte a), b) und c) in beliebiger Reihenfolge erfolgen. Üblicherweise erfolgt die Zugabe von CO jedoch, nachdem die Reaktionspartner in den Schritten a) und b) vorgelegt wurden. Die Schritte c) und d) können gleichzeitig oder nacheinander erfolgen. Darüber hinaus kann CO auch in mehreren Schritten zugeführt werden, so dass beispielsweise zunächst ein Teil des CO zugeführt, dann erwärmt und anschließend ein weiterer Teil CO zugeführt wird.

Das erfindungsgemäße Verfahren sieht vor, dass dem Reaktionsgemisch kein zusätzlicher Alkohol zugesetzt wird. Damit unterscheidet sich das erfindungsgemäße Verfahren entscheidend von den im Stand der Technik bekannten Alkoxycarbonylierungsverfahren, bei denen ein Alkohol als Reaktionspartner eingesetzt wird. Im Rahmen der Erfindung ist indes nicht ausgeschlossen, dass sich im Verlauf der Reaktion ein Alkohol als Neben- oder Zwischenprodukt bildet.

In einer Ausführungsform handelt es sich bei den erfindungsgemäßen Phosphinliganden um Verbindungen gemäß einer der Formeln (**II**) und (**III**)

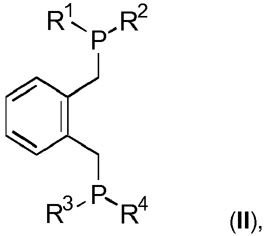

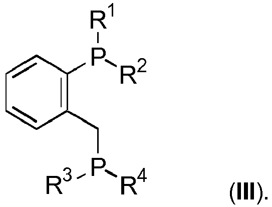

Dabei haben die Reste R¹, R², R³, R⁴ jeweils die oben genannte Bedeutung.

In einer besonders bevorzugten Ausführungsform handelt es sich bei dem erfindungsgemäßen Phosphinliganden um eine Verbindung der Formel (**II**), wobei die Reste R¹, R², R³, R⁴ die oben genannte Bedeutung haben.

Der Begriff (C₁-C₁₂)-Alkyl umfasst geradkettige und verzweigte Alkylgruppen mit 1 bis 12 Kohlenstoffatomen. Vorzugsweise handelt es sich dabei um (C₁-C₃)-Alkylgruppen, besonders bevorzugt (C₁-C₆)-Alkyl, am meisten bevorzugt (C₁-C₄)-Alkyl.

Geeignete (C₁-C₁₂)-Alkylgruppen sind insbesondere Methyl, Ethyl, Propyl, Isopropyl, *n*-Butyl, *iso*-Butyl, *sec*-Butyl, *tert*-Butyl, *n*-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,1-Dimethylbutyl, 1,2-Dimethylbutyl, 2,2-Dimethylbutyl, 1,3-Dimethylbutyl, 2,3-Dimethylbutyl, 3,3-Dimethylbutyl, 1,1,2-Trimethylpropyl, 1,2,2-Trimethylpropyl, 1-Ethylbutyl, 1-Ethyl-2-methylpropyl, *n*-Heptyl, 2-Heptyl, 3-Heptyl, 2-Ethylpentyl, 1-Propylbutyl, *n*-Octyl, 2-Ethylhexyl, 2-Propylheptyl, Nonyl, Decyl.

Die Erläuterungen zum Begriff (C₁-C₁₂)-Alkyl gelten insbesondere auch für die Alkylgruppen in -O-(C₁-C₁₂)-Alkyl, -S-(C₁-C₁₂)-Alkyl, -COO-(C₁-C₁₂)-Alkyl, -CONH-(C₁-C₁₂)-Alkyl, -CO-(C₁-C₁₂)-Alkyl und -N-[(C₁-C₁₂)-Alkyl]₂.

Der Begriff (C₃-C₁₂)-Cycloalkyl umfasst mono-, bi- oder tricyclische Kohlenwasserstoffgruppen mit 3 bis 12 Kohlenstoffatomen. Vorzugsweise handelt es sich dabei um (C₅-C₁₂)-Cycloalkyl.

Die (C₃-C₁₂)-Cycloalkylgruppen weisen vorzugsweise 3 bis 8, besonders bevorzugt 5 oder 6, Ringatome auf.

Geeignete (C₃-C₁₂)-Cycloalkylgruppen sind insbesondere Cyclopropyl, Cyclobutyl, Cyclopentyl, Cyclohexyl, Cycloheptyl, Cyclooctyl, Cyclododecyl, Cyclopentadecyl, Norbonyl, Adamantyl.

Die Erläuterungen zum Begriff (C₃-C₁₂)-Cycloalkyl gelten insbesondere auch für die Cycloalkylgruppen in -O-(C₃-C₁₂)-Cycloalkyl, -S-(C₃-C₁₂)-Cycloalkyl, -COO-(C₃-C₁₂)-Cycloalkyl, -CONH-(C₃-C₁₂)-Cycloalkyl, -CO-(C₃-C₁₂)-Cycloalkyl.

Der Begriff (C₃-C₁₂)-Heterocycloalkyl umfasst nichtaromatische, gesättigte oder teilweise ungesättigte cycloaliphatische Gruppen mit 3 bis 12 Kohlenstoffatomen, wobei eins oder mehrere der Ringkohlenstoffatome durch Heteroatome ersetzt sind. Die (C₃-C₁₂)-Heterocycloalkylgruppen weisen vorzugsweise 3 bis 8, besonders bevorzugt 5 oder 6, Ringatome auf und sind ggf. mit aliphatischen Seitenketten substituiert. In den Heterocycloalkylgruppen sind im Unterschied zu den Cycloalkylgruppen eins oder mehrere der Ringkohlenstoffatome durch Heteroatome oder heteroatomhaltige Gruppen ersetzt. Die Heteroatome oder die heteroatomhaltigen Gruppen sind vorzugsweise ausgewählt unter O, S, N, N(=O), C(=O), S(=O). Eine (C₃-C₁₂)-Heterocycloalkylgruppe im Sinne dieser Erfindung ist somit auch Ethylenoxid.

Geeignete (C₃-C₁₂)-Heterocycloalkylgruppen sind insbesondere Tetrahydrothiophenyl, Tetrahydrofuryl, Tetrahydropyranyl und Dioxanyl.

Der Begriff (C₆-C₂₀)-Aryl umfasst mono- oder polycyclische aromatische Kohlenwasserstoffreste mit 6 bis 20 Kohlenstoffatomen. Vorzugsweise handelt es sich dabei um (C₆-C₁₄)-Aryl, besonders bevorzugt (C₆-C₁₀)-Aryl.

Geeignete (C₆-C₂₀)-Arylgruppen sind insbesondere Phenyl, Naphthyl, Indenyl, Fluorenyl, Anthracenyl, Phenanthrenyl, Naphthacenyl, Chrysenyl, Pyrenyl, Coronenyl. Bevorzugte (C₆-C₂₀)-Arylgruppen sind Phenyl, Naphthyl und Antracenyl.

Der Begriff (C₃-C₂₀)-Heteroaryl umfasst mono- oder polycyclische aromatische Kohlenwasserstoffreste mit 3 bis 20 Kohlenstoffatomen, wobei eins oder mehrere der Kohlenstoffatome durch Heteroatome ersetzt sind. Bevorzugte Heteroatome sind N, O und S. Die (C₃-C₂₀)-Heteroarylgruppen weisen 3 bis 20, bevorzugt 6 bis 14, besonders bevorzugt 6 bis 10 Ringatome auf.

Geeignete (C₃-C₂₀)-Heteroarylgruppen sind insbesondere Furyl, Thienyl, Pyrrolyl, Oxazolyl, Isoxazolyl, Thiazolyl, Isothiazolyl, Imidazolyl, Pyrazolyl, Furazanyl, Tetrazolyl, Pyridyl, Pyridazinyl, Pyrimidyl, Pyrazinyl, Benzofuranyl, Indolyl, Isoindolyl, Benzimidazolyl, Chinolyl, Isochinolyl.

Der Begriff Halogen umfasst insbesondere Fluor, Chlor, Brom und lod. Besonders bevorzugt sind Fluor und Chlor.

In einer Ausführungsform können die Reste R¹, R², R³, R⁴, falls sie für -(C₁-C₁₂)-Alkyl, , -(C₃-C₁₂)-Cycloalkyl, -(C₃-C₁₂)-Heterocycloalkyl, -(C₆-C₂₀)-Aryl, oder -(C₃-C₂₀)-Heteroaryl stehen, jeweils unabhängig voneinander mit einem oder mehreren Substituenten ausgewählt aus -(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -S-(C₁-C₁₂)-Alkyl, -S-(C₃-C₁₂)-Cycloalkyl, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, -COOH, -SO₃H, -NH₂, Halogen substituiert sein.

In einer Ausführungsform können die Reste R¹, R², R³, R⁴, falls sie für -(C₁-C₁₂)-Alkyl, -(C₃-C₁₂)-Cycloalkyl, -(C₃-C₁₂)-Heterocycloalkyl, -(C₆-C₂₀)-Aryl, oder -(C₃-C₂₀)-Heteroaryl stehen, jeweils unabhängig voneinander mit einem oder mehreren Substituenten ausgewählt aus -(C₁-C₁₂)-Alkyl, -(C₃-C₁₂)-Cycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, substituiert sein.

In einer Ausführungsform können die Reste R¹, R², R³, R⁴, falls sie für -(C₁-C₁₂)-Alkyl, -(C₃-C₁₂)-Cycloalkyl, -(C₃-C₁₂)-Heterocycloalkyl, -(C₆-C₂₀)-Aryl oder -(C₃-C₂₀)-Heteroaryl stehen, jeweils unabhängig voneinander mit einem oder mehreren Substituenten ausgewählt aus -(C₁-C₁₂)-Alkyl, -O-(C₁-C₁₂)-Alkyl-(C₆-C₂₀)-Aryl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl substituiert sein.

In einer Ausführungsform können die Reste R¹, R², R³, R⁴, falls sie für -(C₁-C₁₂)-Alkyl, -(C₃-C₁₂)-Cycloalkyl, -(C₃-C₁₂)-Heterocycloalkyl, -(C₆-C₂₀)-Aryl, oder -(C₃-C₂₀)-Heteroaryl stehen, jeweils unabhängig voneinander mit einem oder mehreren Substituenten ausgewählt aus -(C₁-C₁₂)-Alkyl und -(C₃-C₂₀)-Heteroaryl substituiert sein.

In einer Ausführungsform sind die Reste R¹, R², R³, R⁴ unsubstituiert, falls sie für -(C₁-C₁₂)-Alkyl, -(C₃-C₁₂)-Cycloalkyl, oder -(C₃-C₁₂)-Heterocycloalkyl stehen, und können wie beschrieben substituiert sein, falls sie für -(C₆-C₂₀)-Aryl, oder -(C₃-C₂₀)-Heteroaryl stehen.

In einer Ausführungsform sind die Reste R¹, R², R³, R⁴ unsubstituiert, falls sie für -(C₁-C₁₂)-Alkyl, -(C₃-C₁₂)-Cycloalkyl, -(C₃-C₁₂)-Heterocycloalkyl, -(C₆-C₂₀)-Aryl, oder -(C₃-C₂₀)-Heteroaryl stehen.

In einer Ausführungsform sind R¹, R², R³, R⁴ jeweils unabhängig voneinander ausgewählt aus -(C₁-C₁₂)-Alkyl, -(C₆-C₂₀)-Aryl, -(C₃-C₂₀)-Heteroaryl;
wobei mindestens einer der Reste R¹, R², R³, R⁴ für einen -(C₃-C₂₀)-Heteroarylrest steht; und R¹, R², R³, R⁴, falls diese für -(C₁-C₁₂)-Alkyl, -(C₆-C₂₀)-Aryl oder -(C₃-C₂₀)-Heteroaryl stehen, jeweils unabhängig voneinander mit einem oder mehreren der oben beschriebenen Substituenten substituiert sein können.

In einer Ausführungsform stehen mindestens zwei der Reste R¹, R², R³, R⁴ für einen -(C₃-C₂₀)-Heteroarylrest.

In einer Ausführungsform stehen die Reste R¹ und R³ jeweils für einen -(C₃-C₂₀)-Heteroarylrest und können jeweils unabhängig voneinander mit einem oder mehreren der oben beschriebenen Substituenten substituiert sein. Vorzugsweise sind R² und R⁴ dabei unabhängig voneinander ausgewählt aus -(C₁-C₁₂)-Alkyl, -(C₃-C₁₂)-Cycloalkyl, -(C₃-C₁₂)-Heterocycloalkyl, -(C₆-C₂₀)-Aryl, besonders bevorzugt aus -(C₁-C₁₂)-Alkyl, -(C₃-C₁₂)-Cycloalkyl, -(C₆-C₂₀)-Aryl, am meisten bevorzugt aus -(C₁-C₁₂)-Alkyl. R² und R⁴ können dabei unabhängig voneinander mit einem oder mehreren der oben beschriebenen Substituenten substituiert sein.

In einer Ausführungsform stehen die Reste R¹, R², R³ und R⁴ für einen -(C₆-C₂₀)-Heteroarylrest und können jeweils unabhängig voneinander mit einem oder mehreren der oben beschriebenen Substituenten substituiert sein.

In einer Ausführungsform sind die Reste R¹, R², R³ und R⁴, falls sie für einen Heteroarylrest stehen, jeweils unabhängig voneinander ausgewählt aus Heteroarylresten mit fünf bis zehn Ringatomen, vorzugsweise fünf oder sechs Ringatomen.

In einer Ausführungsform stellen die Reste R¹, R², R³ und R⁴, falls sie für einen Heteroarylrest stehen, einen Heteroarylrest mit fünf Ringatomen dar.

In einer Ausführungsform sind die Reste R¹, R², R³ und R⁴, falls sie für einen Heteroarylrest stehen, jeweils unabhängig voneinander ausgewählt aus Heteroarylresten mit sechs bis zehn Ringatomen.

In einer Ausführungsform stellen die Reste R¹, R², R³ und R⁴, falls sie für einen Heteroarylrest stehen, einen Heteroarylrest mit sechs Ringatomen dar.

In einer Ausführungsform sind die Reste R¹, R², R³ und R⁴, falls sie für einen Heteroarylrest stehen, ausgewählt aus Furyl, Thienyl, Pyrrolyl, Oxazolyl, Isoxazolyl, Thiazolyl, Isothiazolyl, Imidazolyl, Pyrazolyl, Furazanyl, Tetrazolyl, Pyridyl, Pyridazinyl, Pyrimidyl, Pyrazinyl, Benzofuranyl, Indolyl, Isoindolyl, Benzimidazolyl, Chinolyl, Isochinolyl, wobei die genannten Heteroarylreste wie oben beschrieben substituiert sein können.

In einer Ausführungsform sind die Reste R¹, R², R³ und R⁴, falls sie für einen Heteroarylrest stehen, ausgewählt aus Furyl, Thienyl, Pyrrolyl, Imidazolyl, Pyridyl, Pyrimidyl, Indolyl, wobei die genannten Heteroarylreste wie oben beschrieben substituiert sein können.

In einer Ausführungsform sind die Reste R¹, R², R³ und R⁴, falls sie für einen Heteroarylrest stehen, ausgewählt aus 2-Furyl, 2-Thienyl, 2-Pyrrolyl, 2-lmidazolyl, 2-Pyridyl, 2-Pyrimidyl, 2-Indolyl, wobei die genannten Heteroarylreste wie oben beschrieben substituiert sein können.

In einer Ausführungsform sind die Reste R¹, R², R³ und R⁴, falls sie für einen Heteroarylrest stehen, ausgewählt aus 2-Furyl, 2-Thienyl, N-Methyl-2-Pyrrolyl, N-Phenyl-2-Pyrrolyl, N-(2-Methoxyphenyl)-2-Pyrrolyl, 2-Pyrrolyl, N-Methyl-2-Imidazolyl, 2-Imidazolyl, 2-Pyridyl, 2-Pyrimidyl, N-Phenyl-2-Indolyl, 2-Indolyl, wobei die genannten Heteroarylreste nicht weiter substituiert sind.

Besonders bevorzugt stellen die Reste R¹, R², R³ und R⁴, falls sie für einen Heteroarylrest stehen, Pyridyl dar, insbesondere 2-Pyridyl.

In einer Ausführungsform stellen R¹ und R³ einen Pyridylrest, bevorzugt 2-Pyridyl, dar und R² und R⁴ stehen für -(C₁-C₁₂)-Alkyl, wobei R¹, R², R³ und R⁴ jeweils wie oben beschrieben substituiert sein können.

In einer Ausführungsform handelt es sich bei dem Phosphinliganden um eine Verbindung gemäß Formel (1):

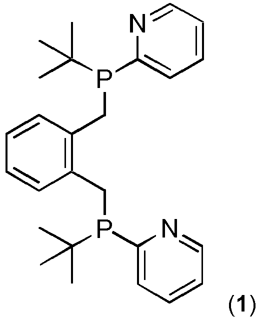

Die in dem erfindungsgemäßen Verfahren in Schritt a) als Edukt eingesetzten Ether umfassen 3 bis 30 Kohlenstoffatome, bevorzugt 3 bis 22 Kohlenstoffatome, besonders bevorzugt 3 bis 12 Kohlenstoffatome. Die Ether können sich von primären, sekundären oder tertiären Alkoholen ableiten. Bei den Ethern kann es ich auch um cyclische Ether handeln.

In einer Ausführungsform sind die Ether acyclisch und leiten sich von einem primären, sekundären oder tertiären Alkohol ab. Vorzugsweise leiten sich die Ether von einem sekundären oder tertiären Alkohol ab. Besonders bevorzugt sind Ether, die sich von einem tertiären Alkohol ableiten.

In einer Ausführungsform ist der Ether eine Verbindung gemäß der Formel **(IV)** 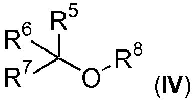
wobei R⁵ ausgewählt ist aus -(C₁-C₁₂)-Alkyl, -(C₃-C₁₂)-Cycloalkyl, -(C₆-C₂₀)-Aryl;
R⁶ und R⁷ jeweils unabhängig voneinander ausgewählt sind aus -H, -(C₁-C₁₂)-Alkyl, -(C₃-C₁₂)-Cycloalkyl, -(C₆-C₂₀)-Aryl;
und R⁸ ausgewählt ist aus -(C₁-C₁₂)-Alkyl, -(C₃-C₁₂)-Cycloalkyl, -(C₆-C₂₀)-Aryl.

In einer bevorzugten Ausführungsform handelt es sich bei R⁵ und R⁸ jeweils um -(C₁-C₁₂)-Alkyl. Vorzugsweise sind R⁵ und R⁸ jeweils ausgewählt aus Methyl, Ethyl, n-Propyl, 2-Propyl, n-Butyl, 2-Butyl, *sec*-Butyl, *tert*-Butyl, n-Pentyl, 2-Pentyl, 3-Pentyl, 2-Methylbutyl, 3-Methylbutyl, 3-Methylbut-2-yl, 2-Methylbut-2-yl, 2,2-Dimethylpropyl. Besonders bevorzugt sind R⁵ und R⁸ jeweils ausgewählt aus Methyl und Ethyl. Am meisten bevorzugt handelt es sich bei R⁵ und R⁸ jeweils um Methyl.

In einer bevorzugten Ausführungsform sind R⁶ und R⁷ jeweils unabhängig voneinander ausgewählt aus -H, -(C₁-C₁₂)-Alkyl und -(C₆-C₂₀)-Aryl. Vorzugsweise sind R⁶ und R⁷ jeweils unabhängig voneinander ausgewählt aus -H, Methyl, Ethyl, n-Propyl, 2-Propyl, n-Butyl, 2-Butyl, *sec*-Butyl, *tert*-Butyl, n-Pentyl, 2-Pentyl, 3-Pentyl, 2-Methylbutyl, 3-Methylbutyl, 3-Methylbut-2-yl, 2-Methylbut-2-yl, 2,2-Dimethylpropyl und Phenyl. Besonders bevorzugt sind R⁶ und R⁷ jeweils unabhängig voneinander ausgewählt aus -H, Methyl, Ethyl, n-Propyl, 2-Propyl, n-Butyl, 2-Butyl, *sec*-Butyl, *tert*-Butyl und Phenyl.

Vorzugsweise stellt höchstens einer der Reste R⁶ und R⁷ -H dar.

In einer alternativen Ausführungsform sind R⁶ und R⁷ jeweils unabhängig voneinander ausgewählt aus -(C₁-C₁₂)-Alkyl und -(C₆-C₂₀)-Aryl. Vorzugsweise sind R⁶ und R⁷ hierbei jeweils unabhängig voneinander ausgewählt aus Methyl, Ethyl, n-Propyl, 2-Propyl, n-Butyl, 2-Butyl, *sec*-Butyl, *tert*-Butyl, n-Pentyl, 2-Pentyl, 3-Pentyl, 2-Methylbutyl, 3-Methylbutyl, 3-Methylbut-2-yl, 2-Methylbut-2-yl, 2,2-Dimethylpropyl und Phenyl. Besonders bevorzugt sind R⁶ und R⁷ hierbei jeweils unabhängig voneinander ausgewählt aus Methyl, Ethyl, n-Propyl, 2-Propyl, n-Butyl, 2-Butyl, *sec*-Butyl, *tert*-Butyl und Phenyl. Insbesondere kann in dieser Ausführungsform R⁵ Methyl sein, wobei R⁶ und R⁷ jeweils unabhängig voneinander ausgewählt sind aus Methyl, tert-Butyl und Phenyl.

In einer bevorzugten Ausführungsform handelt es sich bei dem Ether um Methyl-*tert-*Butylether.

Die erfindungsgemäße Alkoxycarbonylierung wird durch einen Pd-Komplex katalysiert. Der Pd-Komplex kann dabei in Verfahrensschritt b) entweder als präformierter Komplex umfassend Pd und den Phosphinliganden zugegeben werden, oder *in situ* aus einer Verbindung, welche Pd umfasst, und dem freien Phosphinliganden gebildet werden. Dabei wird die Verbindung, welche Pd umfasst, auch als Katalysatorvorstufe bezeichnet.

Die präformierten Komplexe können außerdem weitere Liganden umfassen, die an das Metallatom koordinieren. Dabei handelt es sich beispielsweise um ethylenisch ungesättigte Verbindungen oder Anionen. Geeignete zusätzliche Liganden sind beispielsweise Styrol, Acetat-Anionen, Maleinimide (z.B. N-Methylmaleinimid), 1,4-Naphthochinon, Trifluoroacetat-Anionen oder Chloridanionen.

In dem Fall, dass der Katalysator *in situ* gebildet wird, kann der Ligand im Überschuss zugegeben werden, so dass im Reaktionsgemisch auch ungebundener Ligand vorliegt.

Auch im Falle des Komplexes, der gleich zu Beginn zugesetzt wird, kann zusätzlich weiterer Ligand zugegeben werden, so dass im Reaktionsgemisch auch ungebundener Ligand vorliegt.

In einer Variante ist die Verbindung, welche Pd umfasst, ausgewählt aus Palladiumdichlorid (PdCl₂), Palladium(II)-acetylacetonat [Pd(acac)₂], Palladium(II)-acetat [Pd(OAc)₂], Dichloro(1,5-cyclooctadiene)palladium(II) [Pd(cod)₂Cl₂], Bis(dibenzylideneaceton)palladium [Pd(dba)₂], Bis(acetonitrile)dichloropalladium(II) [Pd(CH₃CN)₂Cl₂], Palladium(cinnamyl)dichlorid [Pd(cinnamyl)Cl₂].

Vorzugsweise handelt es sich bei der Verbindung, welche Pd umfasst, um PdCl₂, Pd(acac)₂ oder Pd(OAc)₂. Besonders geeignet ist Pd(acac)₂.

In einer Variante des Verfahrens wird dem Reaktionsgemisch ein Lösungsmittel zugegeben. Das Lösungsmittelkann dabei beispielsweise ausgewählt sein aus: Toluol, Xylol, Tetrahydrofuran (THF) oder Methylenchlorid (CH₂Cl₂). Vorzugsweise wird dem Reaktionsgemisch Toluol als Lösungsmittel zugegeben.

CO wird in Schritt c) vorzugsweise bei einem CO-Partialdruck zwischen 0,1 und 10 MPa (1 bis 100 bar), bevorzugt zwischen 1 und 8 MPa (10 bis 80 bar), besonders bevorzugt zwischen 2 und 4 MPa (20 bis 40 bar) zugeführt.

Die Reaktionsmischung wird Schritt d) des erfindungsgemäßen Verfahrens vorzugsweise auf eine Temperatur zwischen 10 °C und 180 °C, bevorzugt zwischen 20 und 160 °C, besonders bevorzugt zwischen 40 und 120 °C erwärmt, um den Ether zu einem Ester umzusetzen.

Das Massenverhältnis von Pd zu dem in Schritt a) vorgelegten Ether beträgt vorzugsweise zwischen 0,001 und 0,5 Gew.-%, bevorzugt zwischen 0,01 und 0,1 Gew.-%, besonders bevorzugt zwischen 0,01 und 0,05 Gew.-%.

Das molare Verhältnis des Phosphinliganden zu Pd beträgt vorzugsweise zwischen 0,1:1 und 400:1, bevorzugt zwischen 0,5:1 und 400:1, besonders bevorzugt zwischen 1:1 und 100:1, am meisten bevorzugt zwischen 2:1 und 50:1.

Das Verfahren wird unter Zusatz einer Säure durchgeführt b'): Zugabe einer Säure zum Reaktionsgemisch. Dabei kann es sich vorzugsweise um eine Brønsted- oder eine Lewis-Säure handeln.

Geeignete Brønsted-Säuren haben vorzugsweise eine Säurestärke von pKₛ ≤ 5, bevorzugt eine Säurestärke von pKₛ ≤ 3. Die angegebene Säurestärke pKₛ bezieht sich auf den bei Normalbedingungen (25°C, 1,01325 bar) bestimmten pKₛ-Wert. Bei einer mehrprotonigen Säure bezieht sich die Säurestärke pKₛ im Rahmen dieser Erfindung auf den pKₛ-Wert des ersten Protolyseschrittes.

Vorzugsweise ist die Säure keine Carbonsäure.

Geeignete Brønsted-Säuren sind beispielsweise Perchlorsäure, Schwefelsäure, Phosphorsäure, Methylphosphonsäure und Sulfonsäuren. Vorzugsweise handelt es sich bei der Säure um Schwefelsäure oder eine Sulfonsäure. Geeignete Sulfonsäuren sind beispielsweise Methansulfonsäure, Trifluormethansulfonsäure, tert-Butansulfonsäure, p-Toluolsulfonsäure (PTSA), 2-Hydroxypropan-2-sulfonsäure, 2,4,6-Trimethylbenzolsulfonsäure und Dodecylsulfonsäure. Besonders bevorzugte Säuren sind Schwefelsäure, Methansulfonsäure, Trifluormethansulfonsäure und p-Toluolsulfonsäure.

Als Lewis-Säure kann beispielsweise Aluminiumtriflat eingesetzt werden.

In einer Ausführungsform beträgt die Menge an in Schritt b') zugesetzter Säure 0,3 bis 40 mol-%, bevorzugt 0,4 bis 15 mol-%, besonders bevorzugt 0,5 bis 5 mol-%, am meisten bevorzugt 0,6 bis 4 mol-%, bezogen auf die Stoffmenge des in Schritt a) eingesetzten Ethers.

### Beispiele

Die folgenden Beispiele veranschaulichen die Erfindung.

### Allgemeine Arbeitsvorschriften

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).

Phosphortrichlorid (Aldrich) wurde vor dem Einsatz unter Argon destilliert. Alle präparativen Arbeiten erfolgten in ausgeheizten Gefäßen. Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR_{31P} = SR_{1H} * (BF_{31P} / BF_{1H}) = SR_{1H} * 0,4048. (Robin K. Harris, Edwin D. Becker, Sonia M. Cabral de Menezes, Robin Goodfellow, and Pierre Granger, Pure Appl. Chem., 2001, 73, 1795-1818; Robin K. Harris, Edwin D. Becker, Sonia M. Cabral de Menezes, Pierre Granger, Roy E. Hoffman and Kurt W. Zilm, Pure Appl. Chem., 2008, 80, 59-84).

Die Aufnahme von Kernresonanzspektren erfolgte an Bruker Avance 300 bzw. Bruker Avance 400, die gaschromatografische Analyse an Agilent GC 7890A, die Elementaranalyse an Leco TruSpec CHNS und Varian ICP-OES 715, und die ESI-TOF Massenspektrometrie an Thermo Electron Finnigan MAT 95-XP und Agilent 6890 N/5973 Geräten.

### Darstellung von Chlor-2-pyridyl-tert-butylphosphin (Vorstufe A)

Der Grignard für die Synthese von Chlor-2-pyridyl-t-butylphosphin wird nach der "Knochelmethode" mit Isopropylmagnesiumchlorid dargestellt (Angew. Chem. 2004, 43, 2222-2226). Die Aufarbeitung erfolgt nach der Methode von Budzelaar (Organometallics 1990, 9, 1222-1227).

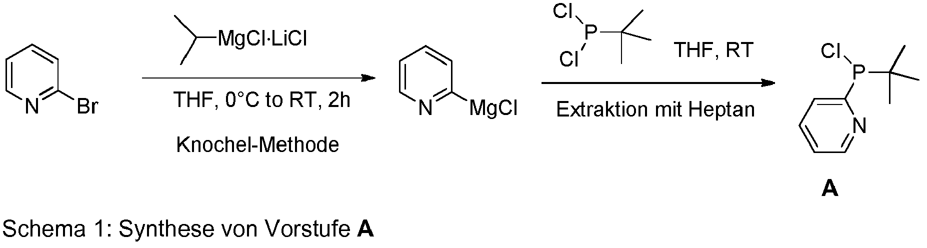

In einen 50 ml-Rundkolben mit Magnetrührer und Septum werden unter Argon 8,07 ml einer 1,3 M Isopropylmagnesium-chloridlösung (Knochel-Reagenz) gegeben und auf -15 °C gekühlt. Danach werden zügig 953.5 µl (10 mmol) 2-Bromopyridin zugetropft. Die Lösung wird sofort gelb. Man lässt auf-10 °C aufwärmen. Der Umsatz der Reaktion wird wie folgt bestimmt: man entnimmt ca. 100 µl Lösung und gibt sie in 1 ml einer gesättigten Ammoniumchloridlösung. Wenn die Lösung "sprudelt", dann hat sich noch nicht viel Grignard gebildet. Die wässrige Lösung wird mit einer Pipette Ether extrahiert und die organische Phase über Na₂SO₄ getrocknet. Von der etherischen Lösung wird ein GC aufgenommen. Wenn sich viel Pyridin gebildet hat im Vergleich zu 2-Bromopyridin, dann hat man hohe Umsätze. Bei -10 °C hat sich wenig umgesetzt. Nach Aufwärmen auf Raumtemperatur und 1-2 stündigen Rühren wird die Reaktionslösung braungelb. Ein GC-Test zeigt vollständigen Umsatz. Jetzt kann die Grignardlösung zu einer Lösung von 1,748 g (11 mmol) Dichloro-tert-butylphosphin in 10 ml THF, die vorher auf -15 °C gekühlt worden ist, mit einer Spritzenpumpe langsam zugetropft werden. Wichtig ist, dass die Dichloro-tert-butylphosphinlösung gekühlt wird. Bei Raumtemperatur würde man erhebliche Mengen an Dipyridyl-tert-butylphosphin erhalten. Es entsteht anfangs eine klare gelbe Lösung, die dann trübe wird. Man lässt auf Raumtemperatur aufwärmen und über Nacht Rühren. Laut GCMS hat sich viel Produkt gebildet. Man entfernt das Lösungsmittel im Hochvakuum und erhält einen weißlichen Feststoff, der braune Stellen enthält. Der Feststoff wird mit 20 ml Heptan suspendiert und der Feststoff wird im Ultraschallbad zerkleinert. Nach dem Absetzen lassen des weißen Feststoffes wird die Lösung dekantiert. Der Vorgang wird zwei Mal mit je 10-20 ml Heptan wiederholt. Nach dem Einengen der Heptanlösung im Hochvakuum wird diese unter Vakuum destilliert. Bei 4,6 mbar, 120 °C Ölbad und 98 °C Übergangstemperatur kann das Produkt destilliert werden. Man erhält 1,08 g eines farblosen Öls. (50%).

Analytische Daten: ¹H NMR (300 MHz, C₆D₆): δ 8,36 (m, 1H, Py), 7,67 (m, 1H, Py), 7,03-6,93 (m, 1H, Py), 6,55-6,46 (m, 1H, Py), 1,07 (d, J = 13,3 Hz, 9H, t-Bu).

¹³C NMR (75 MHz, C₆D₆): δ 162,9, 162,6, 148,8, 135,5, 125,8, 125,7, 122,8, 35,3, 34,8, 25,9 und 25,8.

³¹P NMR (121 MHz, C₆D₆) δ 97,9.

MS (EI) *m:z* (relative Intensität) 201 (M⁺,2), 147(32), 145 (100), 109 (17), 78 (8), 57,1 (17).

### Darstellung von Ligand 1 (α,α'-Bis(2-pyridyl(t-butyl)phosphino)o-xylen)

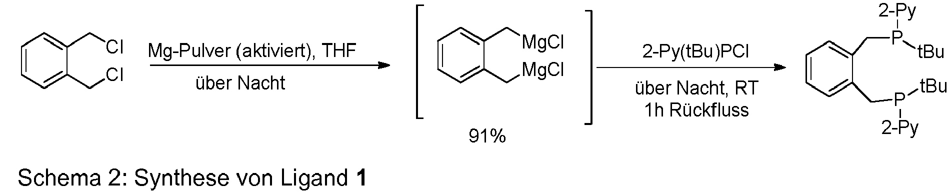

(Lit: Graham Eastham et al., Patent US 6335471)
675 mg (27,8 mmol, 4 eq) Mg-Pulver werden in der Glovebox in einem 250 ml-Rundkolben mit Stickstoffhahn und Magnetrührkern eingewogen und mit einem Septum verschlossen. Man legt an den Rundkolben Hochvakuum (ca. 5x10⁻² mbar) an und erwärmt für 45 Minuten auf 90 °C. Nach Abkühlen auf Raumtemperatur werden 2 Körnchen lod zugegeben und in 20 ml THF gelöst. Man rührt die Suspension ca. 10 Minuten bis die gelbe Farbe des lods verschwunden ist. Nach Absetzen des Magnesiumpulvers wird die trübe THF-Lösung dekantiert und das aktivierte Magnesiumpulver 2 Mal mit 1-2 ml THF gewaschen. Dann werden erneut 20 mL frisches THF zugegeben. Bei Raumtemperatur wird eine Lösung von 1,21 g (6,9 mmol) α,α-Dichloro-o-xylene in 70 ml THF mit der Spritzenpumpe langsam zugetropft. Die THF-Lösung verfärbt sich langsam dunkel. Am nächsten Tag wird die THF-Suspension von dem nicht umgesetzten Magnesiumpulver filtriert und der Gehalt an Grignardverbindung wird wie folgt bestimmt:
1 ml Grignardlösung wird in einer gesättigten wässrigen Lösung von NH₄Cl gequencht und mit Ether extrahiert. Nach Trocknen über Na₂SO₄ wird von der Etherlösung ein GC aufgenommen. Man sieht qualitativ, dass ausschließlich o-Xylen entstanden ist.

Quantitative Bestimmung von dem Gehalt der Grignardlösung:
1 ml Grignardlösung wird mit 2 ml 0,1 M HCL gequenched und die überschüssige Säure mit 0,1 M NaOH titriert. Als Indikator ist eine wässrige 0,04%ige Bromkresollösung geeignet. Farbumschlag geht von gelb nach blau. Es sind 0,74 ml an 0.1 M NaOH verbraucht worden. 2mL-0,74 ml = 1,26 ml, das entspricht 0,126 mmol Grignardverbindung. Da ein Digrignard vorliegt, ist die Grignardlösung 0,063 M. Das sind über 90% Ausbeute.

In einem 250mL Dreihalskolben mit Rückflusskühler und Magnetrührer werden unter Argon 1,8 g (8,66 mmol) Chlorphosphin (2-Py(tBu)PCI) in 10 ml THF gelöst und auf -60 °C gekühlt. Dann werden 55 ml der oben bestimmten Grignardlösung (0,063 M, 3,46 mmol) langsam bei dieser Temperatur mit der Spritzenpumpe zugetropft. Die Lösung bleibt zunächst klar und wird dann intensiv gelb. Nach 1,5 Stunden wird die Lösung trüb. Man lässt über Nacht auf Raumtemperatur aufwärmen und man erhält eine klare gelbe Lösung. Zur Vervollständigung der Reaktion erhitzt man 1 Stunde unter Rückfluss. Nach Abkühlen gibt man 1 ml H₂O dazu und die Lösung entfärbt sich und wird milchig trüb. Nach Entfernen von THF im Hochvakuum erhält man einen zähen, hellgelben Feststoff. Man gibt 10 ml Wasser und 10 ml Ether dazu und erhält zwei homogene klare Phasen, die sich gut trennen lassen. Die wäßrige Phase wird zweimal mit Ether extrahiert. Nach Trocknen der organischen Phase mit Na₂SO₄ wird der Ether im Hochvakuum entfernt und man erhält einen zähen fast farblosen Feststoff. Dieser wird in 5 mL MeOH unter Erwärmen auf dem Wasserbad gelöst und über Celite filtriert. Bei -28 °C erhält man über Nacht 772 mg Produkt in Form von weißen Kristallen. (51%). Aus der Mutterlösung konnten nach Einengen nochmals 100 mg isoliert werden. Die Gesamtausbeute beträgt 57,6 %.

¹H NMR (300 MHz, C₆D₆): δ 8,58 (m, 2H, Py), 7,31-7,30 (m, 2H, Benzol), 7,30-7,22 (m, 2H, Py), 6,85-6,77 (m, 2H, Py), 6,73 (m, 2H, Benzol), 6,57-6,50 (m, 2H, py), 4,33(dd, J = 13,3 und 4,3 Hz, 2H, CH₂), 3,72-3,62 (m, 2H, CH₂), 121 (d, J = 11,8 Hz, 18H, tBu),
¹³C NMR (75 MHz, C₆D₆): δ 161,3, 161,1, 149,6, 137,8, 137,7, 134,5, 133,3, 132,7, 131,4, 131,3, 125,7, 122,9, 30,7, 30,5, 28,2, 28,0, 26,5, 26,4, 26,2, and 26,1.

³¹P NMR (121 MHz, C₆D₆) δ 8,8, EA berechnet für C₂₆H₃₄N₂P₂: C, 71,54; H, 7,85; N, 6,56; P,14,35, gefunden: C, 71,21; H, 7,55; N, 6,56; P, 14,35.

### Herstellung von Methyl 3-methylbutanoat durch Carbonylierung von Methyl-tert-butylether (MTBE)

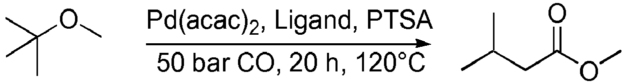

Ein 4 ml-Reaktionsglasgefäß (Vial) wird unter Argon mit Pd(acac)₂ (1,52 mg, 0,25 mol%), PTSA (14,3 mg, 3,75 mol%), **1** (8,7 mg, 1 mol%) und einem Magnetrührer befüllt. Dann werden unter Argon Toluol (2 ml) und MTBE (0,24 mL, 2 mmol) zugesetzt. Dieses Vial wird in einer dafür angefertigten Metallplatte platziert und in einen 300 ml-Autoklav von Parr Instruments überführt. Der Autoklav wird dreimal mit CO gespült und anschließend bei Raumtemperatur mit 50 bar CO befüllt. Die Reaktion wird unter Magnetrührung 20 Stunden bei 120 °C durchgeführt. Anschließend wird auf Raumtemperatur heruntergekühlt und der Autoklav vorsichtig entspannt. Die Ausbeute wurde mittels GC Analyse mit Isooctan (200 µl) als interner Standard durchgeführt (33% Ausbeute an Methyl 3-methylbutanoat).

Dieser Versuch zeigt, dass mit dem erfindungsgemäßen Verfahren Ether durch Reaktion mit CO direkt zu den entsprechenden Estern umgesetzt werden können. Dabei werden unter Verwendung der erfindungsgemäß eingesetzten Liganden signifikante Ausbeuten erzielt. Somit ermöglicht die Erfindung die Verwendung von Ethern an Stelle von ethylenisch ungesättigten Verbindungen als Ausgangsstoff für die Herstellung von Estern.

## Patentansprüche

1. Verfahren umfassend die Verfahrensschritte:
a) Vorlegen eines Ethers mit 3 bis 30 Kohlenstoffatomen;
b) Zugabe eines Phosphinliganden und einer Verbindung, welche Pd umfasst, oder Zugabe eines Komplexes umfassend Pd und einen Phosphinliganden;
b') Zugabe einer Säure zum Reaktionsgemisch;
c) Zuführen von CO;
d) Erwärmen des Reaktionsgemisches, wobei der Ether zu einem Ester umgesetzt wird;
wobei der Phosphinligand eine Verbindung gemäß Formel (**I**) ist 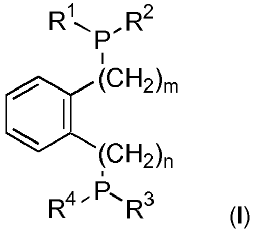 wobei
m und n unabhängig voneinander jeweils 0 oder 1 sind;
R¹, R², R³, R⁴ jeweils unabhängig voneinander ausgewählt sind aus -(C₁-C₁₂)-Alkyl,-(C₃-C₁₂)-Cycloalkyl, -(C₃-C₁₂)-Heterocycloalkyl, -(C₆-C₂₀)-Aryl, -(C₃-C₂₀)-Heteroaryl; mindestens einer der Reste R¹, R², R³, R⁴ für einen -(C₃-C₂₀)-Heteroarylrest steht; und
R¹, R², R³, R⁴, falls diese für -(C₁-C₁₂)-Alkyl, -(C₃-C₁₂)-Cycloalkyl, -(C₃-C₁₂)-Heterocycloalkyl, -(C₆-C₂₀)-Aryl, oder -(C₃-C₂₀)-Heteroaryl stehen, jeweils unabhängig voneinander mit einem oder mehreren Substituenten ausgewählt aus
-(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -S-(C₁-C₁₂)-Alkyl, -S-(C₃-C₁₂)-Cycloalkyl, -COO-(C₁-C₁₂)-Alkyl, -COO-(C₃-C₁₂)-Cycloalkyl, -CONH-(C₁-C₁₂)-Alkyl,-CONH-(C₃-C₁₂)-Cycloalkyl, -CO-(C₁-C₁₂)-Alkyl, -CO-(C₃-C₁₂)-Cycloalkyl, -N-[(C₁-C₁₂)-Alkyl]₂, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl,-(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, -COOH, -SO₃H, -NH₂, Halogen substituiert sein können;
und wobei dem Reaktionsgemisch kein Alkohol zugesetzt wird.

2. Verfahren nach Anspruch 1,
wobei der Phosphinligand eine Verbindung gemäß einer der Formeln (**II**) und (**III**) ist

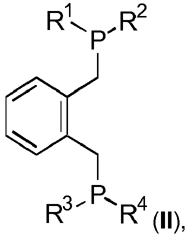

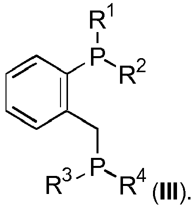

3. Verfahren nach einem der Ansprüche 1 bis 2,
wobei mindestens zwei der Reste R¹, R², R³, R⁴ für einen -(C₃-C₂₀)-Heteroarylrest stehen.

4. Verfahren nach einem der Ansprüche 1 bis 3,
wobei die Reste R¹ und R³ jeweils für einen -(C₃-C₂₀)-Heteroarylrest stehen.

5. Verfahren nach einem der Ansprüche 1 bis 4,
wobei die Reste R¹ und R³ jeweils für einen -(C₃-C₂₀)-Heteroarylrest stehen; und R² und R⁴ jeweils unabhängig voneinander ausgewählt sind aus -(C₁-C₁₂)-Alkyl,-(C₃-C₁₂)-Cycloalkyl, -(C₃-C₁₂)-Heterocycloalkyl, -(C₆-C₂₀)-Aryl.

6. Verfahren nach einem der Ansprüche 1 bis 5,
wobei die Reste R¹ und R³ jeweils für einen -(C₃-C₂₀)-Heteroarylrest stehen; und R² und R⁴ jeweils unabhängig voneinander ausgewählt sind aus -(C₁-C₁₂)-Alkyl.

7. Verfahren nach einem der Ansprüche 1 bis 6,
wobei R¹, R², R³, R⁴, falls diese für einen Heteroarylrest stehen, jeweils unabhängig voneinander ausgewählt sind aus Furyl, Thienyl, Pyrrolyl, Oxazolyl, Isoxazolyl, Thiazolyl, Isothiazolyl, Imidazolyl, Pyrazolyl, Furazanyl, Tetrazolyl, Pyridyl, Pyridazinyl, Pyrimidyl, Pyrazinyl, Benzofuranyl, Indolyl, Isoindolyl, Benzimidazolyl, Chinolyl, Isochinolyl.

8. Verfahren nach einem der Ansprüche 1 bis 7,
wobei der Phosphinligand eine Verbindung gemäß Formel **(1)** ist

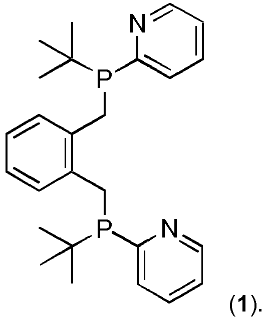

9. Verfahren nach einem der Ansprüche 1 bis 8,
wobei der Ether in Verfahrensschritt a) eine Verbindung gemäß Formel **(IV)** ist 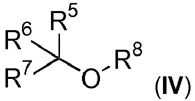
wobei R⁵ ausgewählt ist aus -(C₁-C₁₂)-Alkyl, -(C₃-C₁₂)-Cycloalkyl, -(C₆-C₂₀)-Aryl;
R⁶ und R⁷ jeweils unabhängig voneinander ausgewählt sind aus -H, -(C₁-C₁₂)-Alkyl,-(C₃-C₁₂)-Cycloalkyl, -(C₆-C₂₀)-Aryl;
und R⁸ ausgewählt ist aus -(C₁-C₁₂)-Alkyl, -(C₃-C₁₂)-Cycloalkyl, -(C₆-C₂₀)-Aryl.

10. Verfahren nach Anspruch 9,
wobei R⁵ und R⁸ jeweils -(C₁-C₁₂)-Alkyl sind.

11. Verfahren nach einem der Ansprüche 9 bis 10,
wobei R⁶ und R⁷ jeweils unabhängig voneinander ausgewählt sind aus -H, -(C₁-C₁₂)-Alkyl, -(C₆-C₂₀)-Aryl.

12. Verfahren nach einem der Ansprüche 9 bis 11,
wobei höchstens einer der Reste R⁶ und R⁷ -H ist.

13. Verfahren nach einem der Ansprüche 1 bis 12,
wobei die Verbindung, welche Pd umfasst, in Verfahrenschritt b) ausgewählt ist aus Palladiumdichlorid, Palladium(II)-acetylacetonat, Palladium(II)-acetat, Dichloro(1,5-cyclooctadiene)palladium(II), Bis(dibenzylideneaceton)palladium, Bis(acetonitrile)dichloropalladium(II), Palladium(cinnamyl)dichlorid.

## Claims

1. Process comprising the process steps of:
a) initially charging an ether having from 3 to 30 carbon atoms;
b) adding a phosphine ligand and a compound comprising Pd,
or adding a comprising Pd and a phosphine ligand;
b') adding an acid to the reaction mixture;
c) feeding in CO;
d) heating the reaction mixture, with conversion of the ether; wherein the phosphine ligand is a compound of formula **(I)** 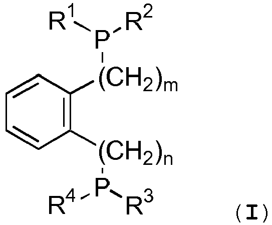 where
m and n are each independently 0 or 1;
R¹, R², R³, R⁴ are each independently selected from -(C₁₋C₁₂)-alkyl, -(C₃-C₁₂)-cycloalkyl, -(C₃-C₁₂)-heterocycloalkyl, -(C₆-C₂₀)-aryl, -(C₃-C₂₀)-heteroaryl;
at least one of the R¹, R², R³, R⁴ radicals is a-(C₃-C₂₀)-heteroaryl radical;
and
R¹, R², R³, R⁴, if they are -(C₁-C₁₂)-alkyl, -(C₃-C₁₂)-cycloalkyl, -(C₃-C₁₂) -heterocycloalkyl, - (C₆-C₂₀)-aryl or -(C₃-C₂₀)-heteroaryl,
may each independently be substituted by one or more substituents selected from
-(C₁-C₁₂)-alkyl, -(C₃-C₁₂) -cycloalkyl, -(C₃-C₁₂)-heterocycloalkyl, -O-(C₁-C₁₂)-alkyl, -O-(C₁-C₁₂)-alkyl- (C₆-C₂₀)-aryl, -O-(C₃-C₁₂) -cycloalkyl, -S-(C₁-C₁₂)-alkyl, -S-(C₃-C₁₂) -cycloalkyl, -COO-(C₁-C₁₂)-alkyl, -COO-(C₃-C₁₂)-cycloalkyl, -CONH-(C₁-C₁₂)-alkyl, -CONH-(C₃-C₁₂)-cycloalkyl, -CO-(C₁-C₁₂)-alkyl, -CO-(C₃-C₁₂)-cycloalkyl, -N-[(C₁-C₁₂)-alkyl]₂, -( C₂₀)-aryl, -(C₆-C₂)-aryl-(C₁-C₁₂)-alkyl, -(C₆-C₂₀)-aryl-O-(C₁-C₁₂)-alkyl, -(C₃-C₂₀)-heteroaryl, -(C₃-C₂₀)-heteroaryl-(C₁-C₁₂)-alkyl, -(C₃-C₂₀)-heteroaryl-O-(C₁-C₁₂)-alkyl, -COOH, -SO₃H, -NH₂, halogen; and wherein no alcohol is added to the reaction mixture.

2. Process according to Claim 1,
wherein the phosphine ligand is a compound of one of the formulae **(II)** and **(III)**

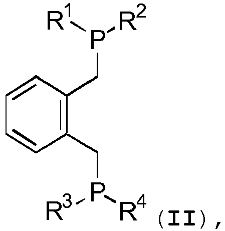

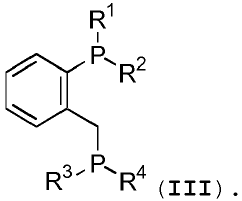

3. Process according to either of Claims 1 and 2, wherein at least two of the R¹, R², R³, R⁴ radicals are a -(C₃-C₂₀)-heteroaryl radical.

4. Process according to any of Claims 1 to 3,
wherein the R¹ and R³ radicals are each a -(C₃-C₂₀)-heteroaryl radical.

5. Process according to any of Claims 1 to 4,
wherein the R¹ and R³ radicals are each a -(C₃-C₂)-heteroaryl radical;
and R² and R⁴ are each independently selected from-(C₁-C₁₂)-alkyl, -(C₃-C₁₂)-cycloalkyl, -(C₃-C₁₂)-heterocycloalkyl, -(C₆-C₂₀)-aryl.

6. Process according to any of Claims 1 to 5,
wherein the R¹ and R³ radicals are each a -(C₃-C₂₀)-heteroaryl radical;
and R² and R⁴ are each independently selected from-(C₁-C₁₂)-alkyl.

7. Process according to any of Claims 1 to 6,
wherein R¹, R², R³, R⁴, if they are a heteroaryl radical, are each independently selected from furyl, thienyl, pyrrolyl, oxazolyl, isoxazolyl, thiazolyl, isothiazolyl, imidazolyl, pyrazolyl, furazanyl, tetrazolyl, pyridyl, pyridazinyl, pyrimidyl, pyrazinyl, benzofuranyl, indolyl, isoindolyl, benzimidazolyl, quinolyl, isoquinolyl.

8. Process according to any of Claims 1 to 7,
wherein the phosphine ligand is a compound of formula **(1)**

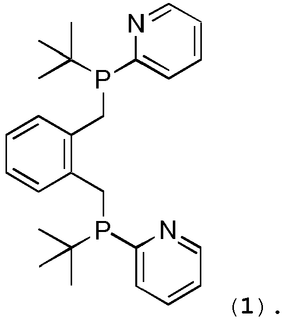

9. Process according to any of Claims 1 to 8,
wherein the ether in process step a) is a compound of formula **(IV)** 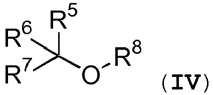
where R⁵ is selected from -(C₁-C₁₂)-alkyl, -(C₃-C₁₂)-cycloalkyl, -(C₆-C₂₀)-aryl;
R⁶ and R⁷ each independently are selected from -H,-(C₁-C₁₂)-alkyl, -(C₃-C₁₂) -cycloalkyl, -(C₆-C₂₀)-aryl; and R⁸ is selected from -(C₁-C₁₂)-alkyl, -(C₃-C₁₂)-cycloalkyl, -(C₆-C₂₀) -aryl.

10. Process according to Claim 9,
wherein R⁵ and R⁸ are each -(C₁-C₁₂)-alkyl.

11. Process according to either of Claims 9 and 10,
wherein R⁶ and R⁷ each independently are selected from -H, -(C₁-C₁₂)-alkyl, -(C₆-C₂₀)-aryl.

12. Process according to any of Claims 9 to 11,
wherein not more than one of the radicals R⁶ and R⁷ is -H.

13. Process according to any of Claims 1 to 12,
wherein the compound comprising Pd in process step b) is selected from palladium dichloride, palladium(II) acetylacetonate, palladium(II) acetate, dichloro(1,5-cyclooctadiene)palladium(II), bis(dibenzylideneacetone)palladium, bis(acetonitrile)dichloropalladium(II), palladium(cinnamyl) dichloride.

## Revendications

1. Procédé comprenant les étapes de procédé suivantes :
a) le chargement d'un éther de 3 à 30 atomes de carbone ;
b) l'ajout d'un ligand phosphine et d'un composé qui comprend du Pd, ou l'ajout d'un complexe comprenant du Pd et un ligand phosphine ;
b') l'ajout d'un acide au mélange réactionnel ;
c) l'introduction de CO ;
d) le chauffage du mélange réactionnel, l'éther étant transformé en un ester ;
le ligand phosphine étant un composé de formule **(I)** 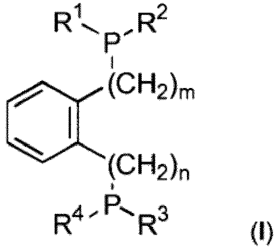 dans laquelle
m et n représentent chacun indépendamment l'un de l'autre 0 ou 1 ;
R¹, R², R³, R⁴ sont chacun choisis indépendamment les uns des autres parmi alkyle en (C₁-C₁₂), cycloalkyle en (C₃-C₁₂), hétérocycloalkyle en (C₃-C₁₂), aryle en (C₆-C₂₀), hétéroaryle en (C₃-C₂₀);
au moins un des radicaux R¹, R², R³, R⁴ représentant un radical hétéroaryle en (C₃-C₂₀);
et
R¹, R², R³, R⁴, si ceux-ci représentent alkyle en (C₁-C₁₂), cycloalkyle en (C₃-C₁₂), hétérocycloalkyle en (C₃-C₁₂), aryle en (C₆-C₂₀) ou hétéroaryle en (C₃-C₂₀), pouvant chacun indépendamment les uns des autres être substitués avec un ou plusieurs substituants choisis parmi :
alkyle en (C₁-C₁₂), cycloalkyle en (C₃-C₁₂), hétérocycloalkyle en (C₃-C₁₂), -O-alkyle en (C₁-C₁₂), -O-alkyle en (C₁-C₁₂)-aryle en (C₆-C₂₀), -O-cycloalkyle en (C₃-C₁₂), -S-alkyle en (C₁-C₁₂), -S-cycloalkyle en (C₃-C₁₂), -COO-alkyle en (C₁-C₁₂), -COO-cycloalkyle en (C₃-C₁₂),-CONH-alkyle en (C₁-C₁₂), -CONH-cycloalkyle en (C₃-C₁₂),-CO-alkyle en (C₁-C₁₂), -CO-cycloalkyle en (C₃-C₁₂), -N-[alkyle en (C₁-C₁₂)]₂, aryle en (C₆-C₂₀), aryle en (C₆-C₂₀)-alkyle en (C₁-C₁₂), aryle en (C₆-C₂₀) -O-alkyle en (C₁-C₁₂), hétéroaryle en (C₃-C₂₀), hétéroaryle en (C₃-C₂₀)-alkyle en (C₁-C₁₂), hétéroaryle en (C₃-C₂₀)-O-alkyle en (C₁-C₁₂),-COOH, -SO₃H, -NH₂, halogène ;
et aucun alcool n'étant ajouté au mélange réactionnel.

2. Procédé selon la revendication 1, dans lequel le ligand phosphine est un composé selon l'une quelconque des formules **(II)** et **(III)**

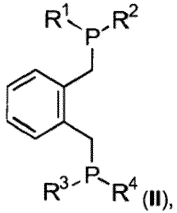

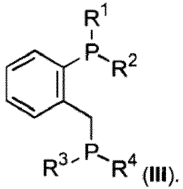

3. Procédé selon l'une quelconque des revendications 1 à 2, dans lequel au moins deux des radicaux R¹, R², R³, R⁴ représentent un radical hétéroaryle en (C₃-C₂₀).

4. Procédé selon l'une quelconque des revendications 1 à 3, dans lequel les radicaux R¹ et R³ représentent chacun un radical hétéroaryle en (C₃-C₂₀).

5. Procédé selon l'une quelconque des revendications 1 à 4, dans lequel les radicaux R¹ et R³ représentent chacun un radical hétéroaryle en (C₃-C₂₀);
et R² et R⁴ sont chacun choisis indépendamment l'un de l'autre parmi alkyle en (C₁-C₁₂), cycloalkyle en (C₃-C₁₂), hétérocycloalkyle en (C₃-C₁₂), aryle en (C₆-C₂₀) .

6. Procédé selon l'une quelconque des revendications 1 à 5, dans lequel les radicaux R¹ et R³ représentent chacun un radical hétéroaryle en (C₃-C₂₀);
et R² et R⁴ sont chacun choisis indépendamment l'un de l'autre parmi alkyle en (C₁-C₁₂).

7. Procédé selon l'une quelconque des revendications 1 à 6, dans lequel R¹, R², R³, R⁴, si ceux-ci représentent un radical hétéroaryle, sont chacun choisis indépendamment les uns des autres parmi furyle, thiényle, pyrrolyle, oxazolyle, isoxazolyle, thiazolyle, isothiazolyle, imidazolyle, pyrazolyle, furazanyle, tétrazolyle, pyridyle, pyridazinyle, pyrimidyle, pyrazinyle, benzofuranyle, indolyle, isoindolyle, benzimidazolyle, quinolyle, isoquinolyle.

8. Procédé selon l'une quelconque des revendications 1 à 7, dans lequel le ligand phosphine est un composé selon la formule **(1)**

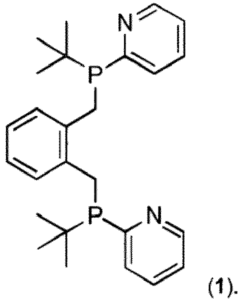

9. Procédé selon l'une quelconque des revendications 1 à 8, dans lequel l'éther à l'étape de procédé a) est un composé selon la formule **(IV)** 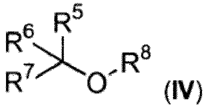
dans laquelle R⁵ est choisi parmi alkyle en (C₁-C₁₂), cycloalkyle en (C₃-C₁₂), aryle en (C₆-C₂₀) ;
R⁶ et R⁷ sont chacun choisis indépendamment l'un de l'autre parmi H, alkyle en (C₁-C₁₂), cycloalkyle en (C₃-C₁₂), aryle en (C₆-C₂₀);
et R⁸ est choisi parmi alkyle en (C₁-C₁₂), cycloalkyle en (C₃-C₁₂), aryle en (C₆-C₂₀).

10. Procédé selon la revendication 9, dans lequel R⁵ et R⁸ représentent chacun alkyle en (C₁-C₁₂).

11. Procédé selon l'une quelconque des revendications 9 à 10, dans lequel R⁶ et R⁷ sont chacun choisis indépendamment l'un de l'autre parmi H, alkyle en (C₁-C₁₂), aryle en (C₆-C₂₀).

12. Procédé selon l'une quelconque des revendications 9 à 11, dans lequel au plus un des radicaux R⁶ et R⁷ représente H.

13. Procédé selon l'une quelconque des revendications 1 à 12, dans lequel le composé qui comprend du Pd à l'étape de procédé b) est choisi parmi le dichlorure de palladium, l'acétylacétonate de palladium (II), l'acétate de palladium (II), le dichloro(1,5-cyclooctadiène)palladium (II), le bis(dibenzylidène-acétone)palladium, le bis(acétonitrile)dichloropalladium (II), le dichlorure de palladium(cinnamyle).
